# EUROPEAN PATENT APPLICATION

(11) **EP 2 363 085 A1**
(43) Date of publication of application: **07.09.2011**
(21) Application number: 09818823.8
(22) Date of filing: 25.09.2009
(51) Int. Cl.: A61B 17/50

(54) **SPATULAS COUNTERING JELLYFISH STINGS**

(30) Priority: 09.10.2008 ES 200802869
(71) Applicant: Torres Ribas, Antonio, Ibiza (Islas Baleares) (ES)
(72) Inventor: Torres Ribas, Antonio, Ibiza (Islas Baleares) (ES)
(86) International application number: PCT/ES2009/000471
(87) International publication number: WO 2010/040869

(57) **Abstract**

Constituted from a hard plastic material or similar and designed having an ovoid form, of size such that it may be manipulated with one hand, **characterised in that** it presents a straight lowered edge. At approximately the middle of the spatula a prominence traverses it horizontally in the form of a stop. Commencing therefrom the two faces of the surface of the spatula rise in height to the extremity continuing with the same thickness as the prominence or stop. On the reverse of the aforementioned spatula the zone at the greatest height is manufactured such as to present rugosity.

## Description

The present invention relates to a spatula, blade, spoon, slice or any other tool that is manufactured and specially designed to remove the jellyfish nematocysts or threads. The part which will be in direct contact with the skin should be a minimum thickness to have a close shaving and avoid having any cant that can be dangerous. Its shape is indistinct and may be any geometric shape or even designed just like any shape similarity. This tool can be completely flat and without a handle or with handle like a razor blade. The surface is presented in two height levels and the object of such utensil is extracting a way of shaving the jellyfish nematocysts or threads that are embedded in the human skin at the time of contact of its skin with the tentacles of animals such as jellyfish.

With more than ten thousand species in oceans and seas, jellyfish are responsible for some of the most common human poisonings. They have tentacles made of stinging cells or nematocysts that they use to capture preys or a way of defense. These cells have a capsule with a rolled thread inside the same that when a prey contacts with these nematocysts are opened and the threads are ejected and prodded injecting its venom. These are common on the shores of the beaches and swimmers are frequent victims of the jellyfish tentacles as well as the professional fishermen.

It falls within the field of sanitary accessories for shaving the skin and more concretely in those intended for shaving jellyfish threads.

Thus, only are known utensils instead of developed for this purpose its use is derived for such necessities and that is because to the beaches are directed to people in order to spend a nice day and they have not in mind the stings of these animals. That is why it can see swimmers with credit cards which are flat and have rigid and thin edges using these to separate the thread remains of their skin or with knifes or pocket-knifes. Until now the process is as follows: the jellyfish remains are removed always with gloves and the skin is rinsed with salt water, a solution is applied with acid acetic or alcohol at five percent, then is shaved with a razor blade and soap or is scraped with a knife, is dried, and a hydrocortisone ointment is applied.

U0006710 describes a spatula to remove skin rashes and pimples.

What is known in the prior art have these problems:
- The use for scraping with credit cards is not hygienic because the edge of these is used in many ATMs and collection bands or knuckle busters that can adhere to the card any kind of dirt.
- The fact of using the knife or pocket-knife in order to root out the threads and/or bits of the tentacles can bring even worse consequences since it can be overextended with it suffering injuries attached with its fine edge.

- The knife or the pocket-knife neither is unhygienic because its sharp blade may be attached dirt due to the use in other necessities.
- Both the credit card and razor blade or pocket-knife allow that the threads extracted from the skin can slide by them coming into contact with the skin of the hand that manipulates said card or razor blade.
- U0006710 describes a spatula to remove pimples from the skin, not animal stings.

Against these problems the invention proposed here presents the following advantages:
- A prominence in the middle of the spatula prevents the sliding to the fingers of the threads extracted from the skin and jellyfish remains.
- It is absolutely hygienic because it possesses only the use of cleaning the skin is preserved only and exclusively for that necessity.
- The dimensions allow carry it in a pocket or small handbag.
- It's easy and inexpensive of manufacture.
- It eliminates having to bring alcohol or acetic acid with all the disadvantages that entails.
- It eliminates the use of the razor blade and the danger of surpassing at the time of attempting to clean the skin.

Thus, the present invention is constituted from a spatula made of a hard plastic material or similar and designed having an ovoid form, of size such that it may be manipulated with one hand that it presents a straight lowered edge in order to put pressure on the skin of the sufferer enough to draw out the attached threads that remain after the jellyfish sting. At approximately the middle of the spatula a prominence traverses it horizontally in the form of a stop so that such remains of threads do not slide to the fingers that handle the spatula. Said remains are trapped in the cited prominence or stop, being thus very easy the cleaning of the spatula. From the stop the two faces of the surface of the spatula rise in height to the extremity continuing with the same thickness as the prominence or stop. On the reverse of the aforementioned spatula the zone at the greatest height is manufactured such as to present rugosity so that the blade, due to is wet, does not slide through in between the fingers and can be gripped strongly.

A different embodiment is constituted from the incorporation of a handle on the back of the piece in order to facilitate its manipulation.

Alternatively it can be designed in a different way provided that it is appropriate to the circumstances.

For a better understanding of what is described in the present specification is accompanied by drawings which by way of non-limitative example is depicted a practical embodiment.

In such drawings:

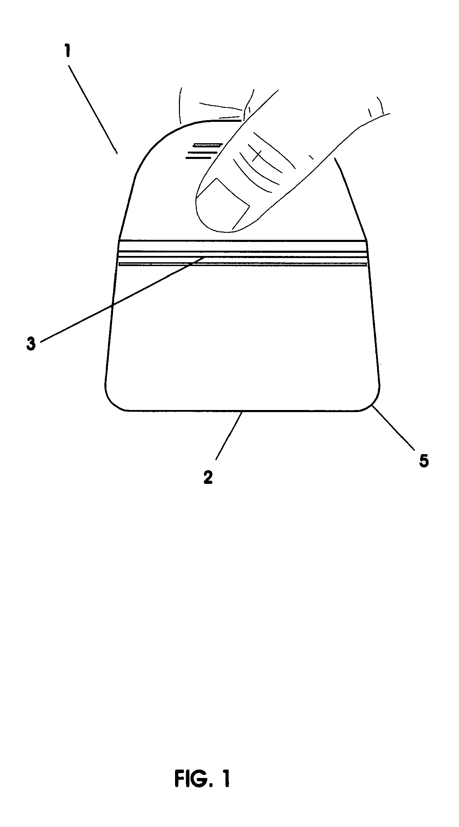

- Figure 1: Plan view of the spatula obverse.

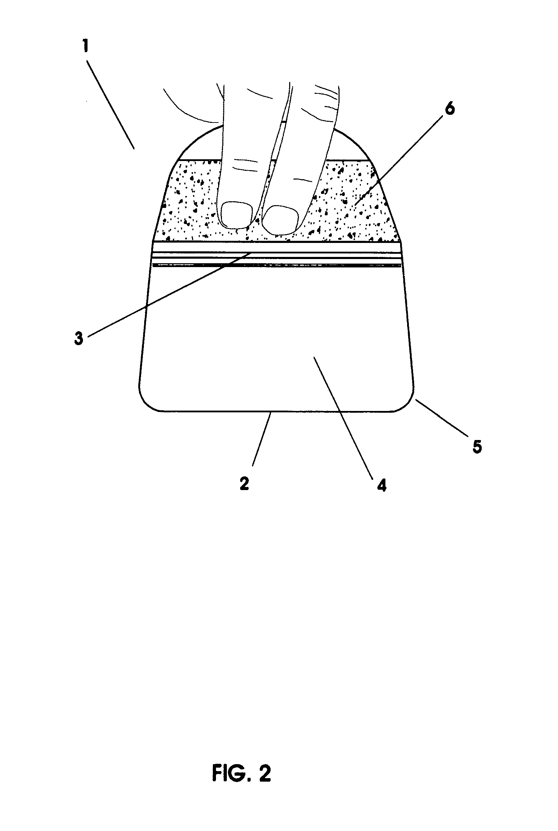

- Figure 2: Plan view of the spatula reverse.

The numbered details correspond to:
1) Spatula
2) Straight edge
3) Prominence
4) Spatula reverse
5) Spatula faces
6) Zone at the greatest height of the spatula

A preferred embodiment is constituted from a spatula (1) made of a hard plastic material or similar and designed having an ovoid form of size such that it may be manipulated with one hand that it presents a straight lowered edge (2). At approximately the middle of the spatula (1) a prominence (3) traverses it horizontally in the form of a stop. Commencing therefrom (3), the two faces (5) of the surface of the spatula (1) rise in height to the extremity continuing with the same thickness as the prominence (3) or stop. On the reverse of the aforementioned spatula (1) the zone at the greatest height (6) is manufactured such as to present rugosity.

## Claims

1. Spatula countering jellyfish stings constituted from a hard plastic material or similar and designed having an ovoid form, of size such that it may be manipulated with one hand, **characterised in that** it presents a straight lowered edge. At approximately the middle of the spatula a prominence traverses it horizontally in the form of a stop. Commencing therefrom the two faces of the surface of the spatula rise in height to the extremity continuing with the same thickness as the prominence or stop. On the reverse of the aforementioned spatula the zone at the greatest height is manufactured such as to present rugosity.

2. Spatula countering jellyfish stings, according to claim 1 **characterised** from the incorporation of a handle on the back of the piece.

3. Spatula countering jellyfish stings, according to claim 1, **characterised in that** it can be designed in a different way provided that it is appropriate to the circumstances.
